(19) Europäisches Patentamt
European Patent Office
Office européen des brevets

(11) Veröffentlichungsnummer: **0 624 593 A2**

# (12) EUROPÄISCHE PATENTANMELDUNG

(21) Anmeldenummer: **94106844.7**

(22) Anmeldetag: **02.05.94**

(51) Int. Cl.5: **C07J 9/00**, C07J 41/00, A61K 31/575

(30) Priorität: **08.05.93 DE 4315389**

(43) Veröffentlichungstag der Anmeldung: **17.11.94 Patentblatt 94/46**

(84) Benannte Vertragsstaaten: **AT BE CH DE DK ES FR GB GR IE IT LI LU NL PT SE**

(71) Anmelder: **HOECHST AKTIENGESELLSCHAFT**
**Brüningstrasse 50**
**D-65929 Frankfurt am Main (DE)**

(72) Erfinder: **Wess, Günther, Dr.**
**Langenselbolder Weg 35**
**D-63526 Erlensee (DE)**
Erfinder: **Enhsen, Alfons, Dr.**
**Odenwaldstrasse 19**
**D-64572 Büttelborn (DE)**
Erfinder: **Glombik, Heiner, Dr.**
**Südhang 16**
**D-65719 Hofheim am Taunus (DE)**
Erfinder: **Kramer, Werner, Dr. Dr.**
**Henry-Moisand-Strasse 19**
**D-55130 Mainz (DE)**

(54) **Gallensäurederivate, Verfahren zu ihrer Herstellung und Verwendung dieser Verbindungen als Arzneimittel.**

(57) Es werden Gallensäurederivate der Formel I

G1 - X - G2 (I)

worin X die angegebene Bedeutung hat,
G1 einen Rest der Formel II,

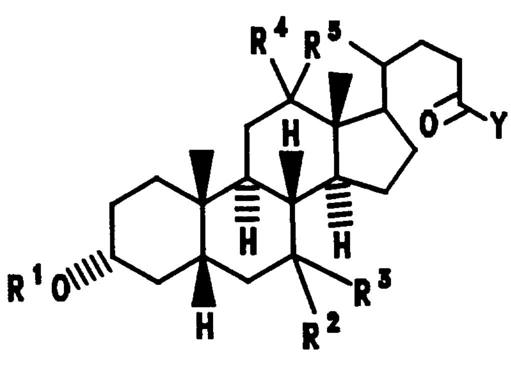

(II)

und
G2 einen Rest der Formel IV

EP 0 624 593 A2

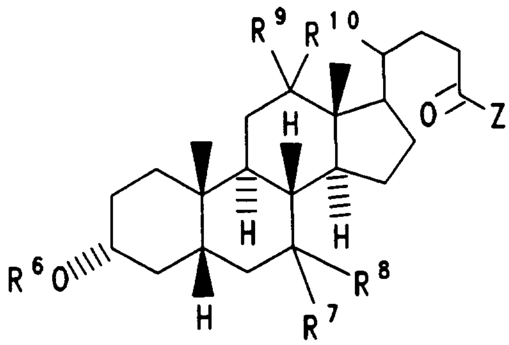

(IV)

bedeutet, wobei die in den Formeln II und IV vorkommenden Reste $R^1$ bis $R^9$, Y und Z die angegebenen Bedeutungen haben, sowie Verfahren zur Herstellung dieser Verbindungen beschrieben. Die Gallensäurederivate der Formel I besitzen wertvolle pharmakologische Eigenschaften und können daher als Arzneimittel verwendet werden.

Die Erfindung betrifft neue Gallensäurederivate, Verfahren zu ihrer Herstellung, pharmazeutische Präparate auf Basis dieser Verbindungen sowie die Verwendung der Gallensäurederivate als Arzneimittel.

Gallensäuren haben eine wichtige physiologische Funktion bei der Fettverdauung, z.B. als Cofaktoren der pankreatischen Lipasen und als natürliche Detergentien zur Solubilisierung von Fetten und fettlöslichen Vitaminen. Als Endprodukt des Cholesterinstoffwechsels werden sie in der Leber synthetisiert, in der Gallenblase gespeichert und aus dieser durch Kontraktion in den Dünndarm abgegeben, wo sie ihre physiologische Wirkung entfalten. Der größte Teil der sezernierten Gallensäuren wird über den enterohepatischen Kreislauf zurückgewonnen. Sie gelangen über die Mesenterialvenen des Dünndarms und das Pfortadersystem wieder zur Leber zurück. Bei der Rückresorption im Darm spielen sowohl aktive als auch passive Transportprozesse eine Rolle. Die Hauptmenge der Gallensäuren wird am Ende des Dünndarms, dem terminalen Ileum, durch ein spezifisches $Na^+$-abhängiges Transportsystem rückresorbiert und gelangt mit dem Mesenterialvenenblut über die Pfortader zurück zur Leber, um von den Leberzellen erneut in die Galle sezerniert zu werden. Im enterohepatischen Kreislauf treten die Gallensäuren sowohl als freie Säuren als auch in Form von Glycin- und Taurinkonjugaten in Erscheinung.

Nichtresorbierbare, unlösliche, basische, vernetzte Polymere werden seit geraumer Zeit zur Bindung von Gallensäuren verwendet und aufgrund dieser Eigenschaften therapeutisch genutzt. In der Patentanmeldung EP-A-0 489 423 beschriebene Gallensäurederivate besitzen eine hohe Affinität zum intestinalen Gallensäuretransportsystem und erlauben daher eine spezifische Hemmung des enterohepatischen Kreislaufs. Als Therapieobjekt werden alle Erkrankungen, bei denen eine Hemmung der Gallensäureresorption im Darm, insbesondere im Dünndarm, wünschenswert erscheint, angesehen. Beispielsweise werden die cholagene Diarrhoe nach Ileumresektion, oder auch erhöhte CholesterinBlutspiegel auf diese Weise behandelt. Im Falle des erhöhten Cholesterin-Blutspiegels kann durch den Eingriff in den enterohepatischen Kreislauf eine Senkung dieses Spiegels erreicht werden. Durch Senkung des im enterohepatischen Kreislauf befindlichen Gallensäurepools wird die entsprechende Neusynthese von Gallensäuren aus Cholesterin in der Leber erzwungen. Zur Deckung des Cholesterinbedarfs in der Leber wird auf das im Blutkreislauf befindliche LDL-Cholesterin zurückgegriffen, wobei die hepatischen LDL-Rezeptoren vermehrt zum Einsatz kommen. Die so erfolgte Beschleunigung des LDL-Katabolismus wirkt sich durch Herabsetzung des atherogenen Cholesterinanteils im Blut aus.

Es bestand die Aufgabe, neue Arzneimittel zu finden, die in der Lage sind, den atherogenen Cholesterinanteil im Blut herabzusetzen bzw. den enterohepatischen Kreislauf hinsichtlich erhöhter Gallensäureausscheidung und daraus folgender Senkung des Cholesterinspiegels zu beeinflussen.

Diese Aufgabe wird durch die erfindungsgemäßen Gallensäurederivate gelöst.

EP-A-0 489 423 betrifft dimere Gallensäurederivate der Formel

G1-X-G2

wobei die Verknüpfung von G1 und G2 in Positionen 3, 7, 12 oder über die Seitenkette über den Linker X erfolgt. Gallensäurederivate, wobei G1 über die Positionen 7 oder 12 und G2 über die Positionen 3, 7, 12 oder die Seitenkette mit X verbunden sind, werden in den Beispielen der genannten europäischen Patentanmeldung nicht beschrieben.

Die Erfindung betrifft daher Gallensäurederivate der Formel I

G1 - X - G2 I

in der G1 einen Rest der Formel II bedeutet,

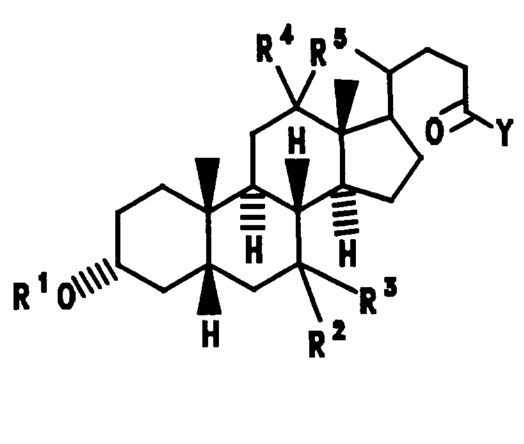

(II)

wobei

Y die folgende Bedeutung hat: OKa, wobei Ka Alkali-, Erdalkali-oder ein quartäres Ammoniumion bedeutet
-OL, -NHL, $-NL_2$,
eine über die Aminogruppe gebundene Aminosäure oder Aminosulfonsäure, wie z.B.
$-NHCH_2COOH$, $-NHCH_2CH_2SO_3H$,

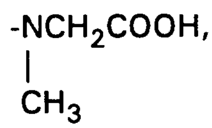, 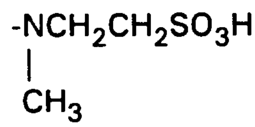

und deren $(C_1$-$C_4)$-Alkylester, Alkali- und Erdalkalisalze sowie quartäre Ammoniumsalze, und wobei L
H, einen Alkyl- oder Alkenylrest mit bis zu 10 C-Atomen, der verzweigt oder unverzweigt ist, einen Cycloalkylrest mit 3 bis 8 C-Atomen, einen Phenyl- oder Benzylrest, die unsubstituiert oder 1- bis 3-fach substituiert sind mit F, Cl, Br, $(C_1$-$C_4)$-Alkyl oder $(C_1$-$C_4)$-Alkoxy,

$R^1$ H, einen Alkyl- oder Alkenylrest mit bis zu 10 C-Atomen, der verzweigt oder unverzweigt ist, einen Cycloalkylrest mit 3 bis 8 C-Atomen,
einen Benzylrest,
einen Biphenylmethyl- oder einen Triphenylmethylrest,
bei denen die Kerne unsubstituiert oder 1- bis 3-fach mit F, Cl, Br, $(C_1$-$C_4)$-Alkyl, $(C_1$-$C_4)$-Alkoxy substituiert sind,
einen Rest

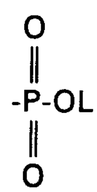, 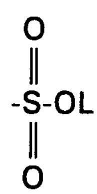 oder 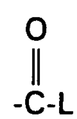

wobei L die oben angegebene Bedeutung hat,

$R^2$ bis $R^5$ wobei $R^2$ und $R^3$ bzw. $R^4$ und $R^5$ jeweils gemeinsam der Sauerstoff einer Carbonylgruppe oder einzeln und jeweils unabhängig voneinander
H, -OT, -ST, -NHT,

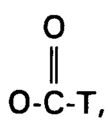, 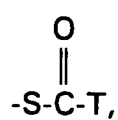, 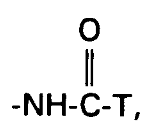, 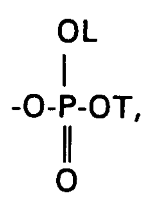, 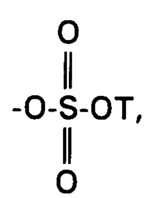,

-T wobei T die Bedeutung von L hat oder eine freie Valenz zur Bindung der Gruppe X aufweist,
wobei insgesamt von G1 nur eine freie Valenz zur Bindung der Gruppe X ausgeht,

X eine Einfachbindung oder eine Gruppe der Formel III ist

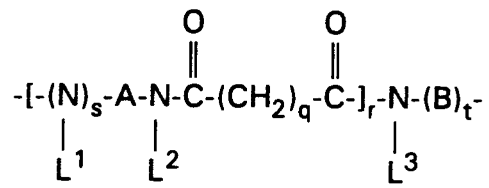

(III),

wobei

A und B — Alkylenketten, die verzweigt oder unverzweigt sind, wobei die Ketten gegebenenfalls durch -O- oder -S- unterbrochen sein können,
$L^1$, $L^2$, $L^3$ gleich oder verschieden sind und die Bedeutung von L haben sowie

q — null bis 5
r — null oder 1
s — null oder 1
t — null oder 1 bedeuten
G2 — einen Rest der Formel IV bedeutet

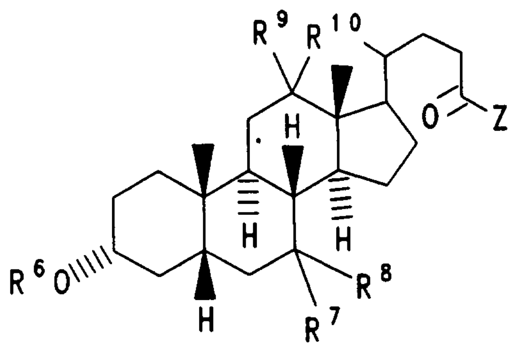

( I V )

wobei

Z — eine freie Valenz zur Gruppe X ist oder die unter Y angegebene Bedeutung hat,
$R^6$ — eine freie Valenz zur Gruppe X ist oder die unter $R^1$ angegebene Bedeutung hat,
$R^7$ bis $R^{10}$ — die unter $R^2$ bis $R^5$ angegebene Bedeutung haben, wobei insgesamt von G2 nur eine freie Valenz zur Gruppe X ausgeht.

Die erfindungsgemäßen Verbindungen besitzen eine hohe Affinität zum spezifischen Gallensäuretransportsystem des Dünndarms und hemmen die Gallensäureresorption in konzentrationsabhängiger und kompetitiver Weise. Weiterhin werden die erfindungsgemäßen Verbindungen selbst nicht resorbiert und gelangen somit nicht in den Blutkreislauf. Durch Anwendung dieses Wirkprinzips kann der enterohepatische Kreislauf sehr spezifisch und effizient unterbrochen werden.

Durch Verwendung der erfindungsgemäßen Verbindungen ist es möglich, die Menge der im enterohepatischen Kreislauf befindlichen Gallensäuren zu vermindern, so daß eine Senkung des Cholesterinspiegels im Serum erfolgt. Avitaminosen sind bei der Anwendung ebenso wenig zu erwarten wie die Beeinflussung der Resorption anderer Arzneimittel oder auch eine negative Wirkung auf die Darmflora. Weiterhin werden die von den Polymeren her bekannten Nebenwirkungen (Obstipation, Steratorrhoe) nicht beobachtet, d.h. die Fettverdauung wird nicht negativ beeinflußt. Wegen der hohen Affinität zum spezifischen Gallensäuretransportsystem des Dünndarms kommt man mit geringen Tagesdosen aus, so daß die Akzeptanz solcher Arzneimittel bei Arzt und Patient sehr hoch sein wird.

Besonders bevorzugt sind Verbindungen der Formel I, in der G1 einen Rest der Formel II bedeutet

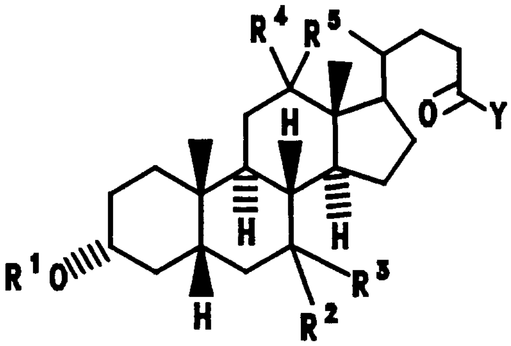

(II)

wobei

Y — OH, O-($C_1$-$C_4$)-Alkyl, $-NHCH_2COOH$,

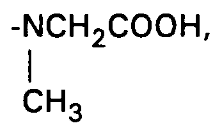

$-NHCH_2CH_2SO_3H$,

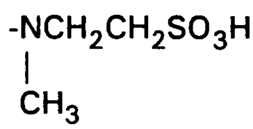

$R^1$ — H, Benzyl, Biphenylmethyl, Formyl, Acetyl,

$R^2$ bis $R^5$ — wobei $R^2$ und $R^3$ bzw. $R^4$ und $R^5$ jeweils gemeinsam der Sauerstoff einer Carbonylgruppe oder einzeln und jeweils unabhängig voneinander H, -OT, -NHT,

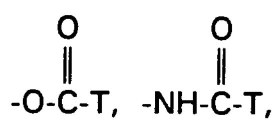

-T bedeuten wobei T H, ein verzweigter oder unverzweigter ($C_1$-$C_4$)-Alkylrest oder eine freie Valenz zur Brückengruppe X darstellt, wobei von G1 insgesamt eine freie Valenz zur Bindung der Gruppe X ausgeht,

X — eine Bindung

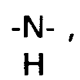

$-CH_2CH_2NH-$

$-CH_2CH_2CH_2NH-$mit

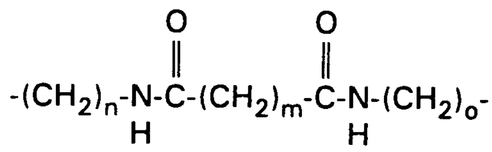

n = 2 oder 3, m = 1 bis 4, o = 2 oder 3 ist,

G2 einen Rest der Formel IV bedeutet

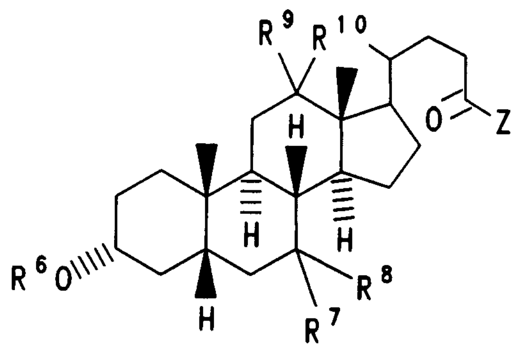

(IV)

wobei

Z eine freie Valenz zur Gruppe X ist oder die vorstehend unter Y angegebene Bedeutung hat,

$R^6$ eine freie Valenz zur Gruppe X ist oder die vorstehend unter $R^1$ angegebene Bedeutung hat,

$R^7$ bis $R^{10}$ die vorstehend unter $R^2$ bis $R^5$ angegebene Bedeutung haben, wobei von G2 nur eine freie Valenz zur Gruppe X ausgeht.

Die Erfindung betrifft ferner ein Verfahren zur Herstellung von Verbindungen der Formel I, das dadurch gekennzeichnet ist, daß man

a) im Falle von X = Einfachbindung geeignete Formen von G1 und G2 nach im Prinzip bekannten Verfahren miteinander zur Reaktion bringt oder

b) im Falle von X = Brückengruppe

α) reaktionsfähige Formen von G1-X mit G2 bzw.

β) reaktionsfähige Formen von G2-X mit G1

nach im Prinzip bekannten Verfahren miteinander zur Reaktion bringt oder

c) aus G1-X1 und X2-G2 nach bekannten oder soweit nicht bekannt, nach den unten näher beschriebenen Verfahren, Verbindungen der allgemeinen Formel I (G1-X-G2) herstellt, wobei X aus X1 und X2 entsteht durch Ausbildung einer kovalenten Bindung, insbesondere innerhalb einer Kondensations- oder Substitutionsreaktion.

a) X = Einfachbindung

Die Gallensäuren G1 werden entweder in freier Form oder in geschützter Form eingesetzt. Nach der Verknüpfung mit G2, die ebenfalls in freier oder geschützter Form vorliegt, erfolgt gegebenenfalls die Abspaltung der Schutzgruppen und gegebenenfalls die Umwandlung der C-24 Carboxylfunktion in ein Derivat. Als Schutzgruppen für die Alkoholgruppen eignen sich zweckmäßigerweise Formyl, Acetyl, Tetrahydropyranyl oder t-Butyldimethylsilyl. Als Schutzgruppen für die C-24 Carboxylgruppe kommen verschiedene Alkyl oder Benzylester in Frage, aber auch z.B. Orthoester.

Beispielsweise reagiert Gallensäure bevorzugt an Position 3, aber auch an Position 7 mit aktivierten Formen von Carbonsäuren, wie Säurechloriden oder gemischten Anhydriden unter Zusatz von Basen wie Trialkylamin, Pyridin, aber auch NaOH bei Zimmertemperatur in geeigneten Lösungsmitteln wie Tetrahydrofuran, Methylenchlorid oder Essigester, aber auch Dimethylformamid (DMF) oder Dimethoxyethan (DME).

Die verschiedenen Isomeren können z.B. chromatographisch getrennt werden. Durch die Verwendung von geeigneten Schutzgruppen läßt sich die Reaktion selektiv durchführen.

Analog lassen sich entsprechende Aminogallensäuren in entsprechende Amide überführen. Auch hier kann die Reaktion entweder mit geschützten oder freien Gallensäuren durchgeführt werden.

Analog lassen sich weitere erfindungsgemäße Verbindungen nach bekannten Standardverfahren verknüpfen.

b) X = eine Brückengruppe

Die unter a) angegebenen Verfahren werden auch angewendet, um die Verknüpfung G1-X mit G2 bzw. G1 mit X-G2 durchzuführen. Zweckmäßigerweise setzt man auch hier den Gallensäureteil entweder geschützt oder ungeschützt ein.

Ein bevorzugtes Herstellungsverfahren besteht darin, reaktionsfähige Formen von G1 mit reaktionsfähigen Formen X-G2 umzusetzen. Gegebenenfalls schließen sich nach der Verknüpfung die Abspaltung von Schutzgruppen und die Umwandlung von C-24 Carboxyl in Derivate an.

Die Herstellung von reaktionsfähigen Gallensäurebausteinen G1-X bzw. X-G2 ist in folgendem Formelschema angegeben.

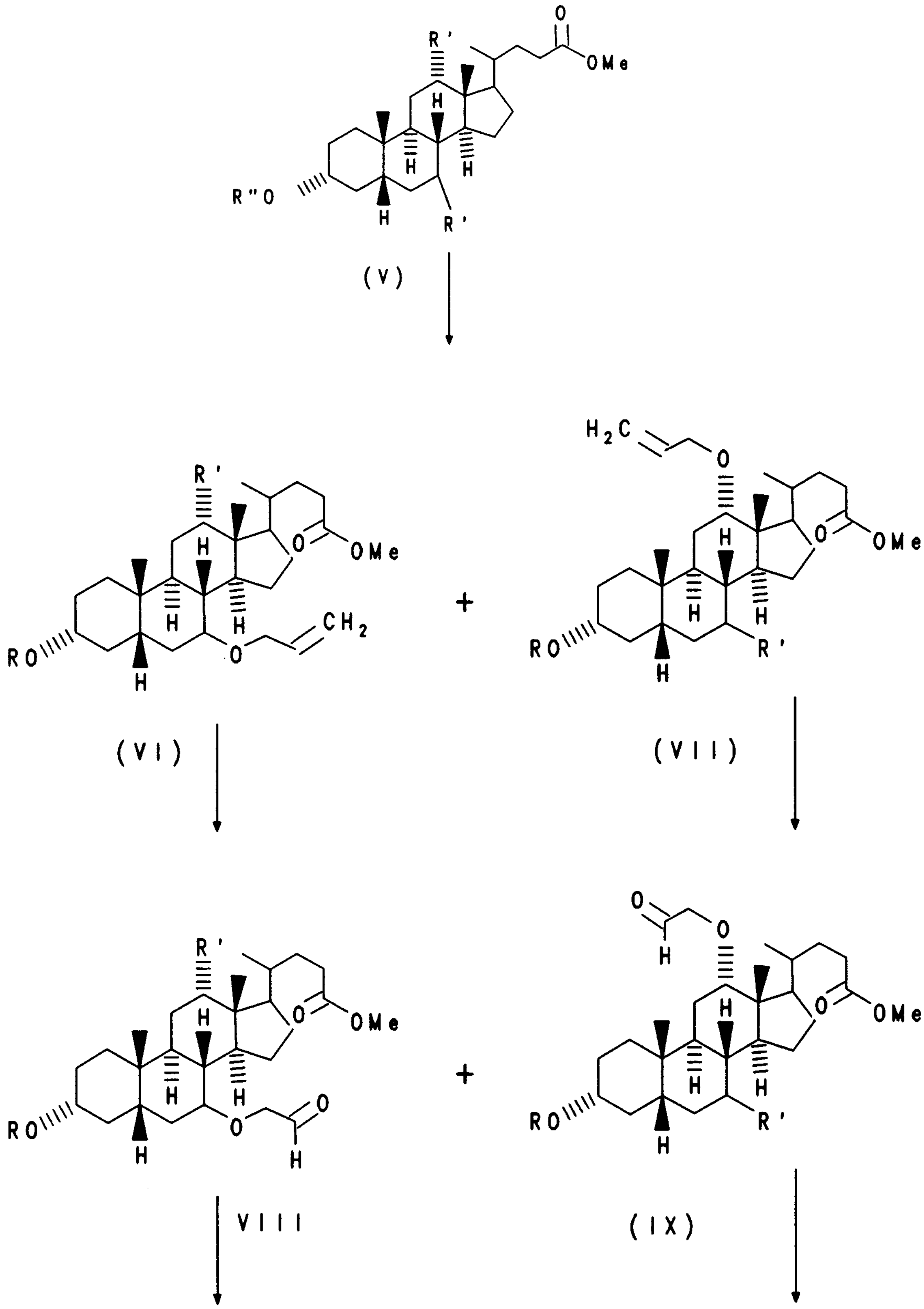

(V)

(VI) + (VII)

VIII + (IX)

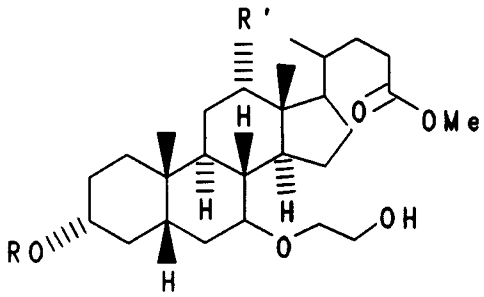

+

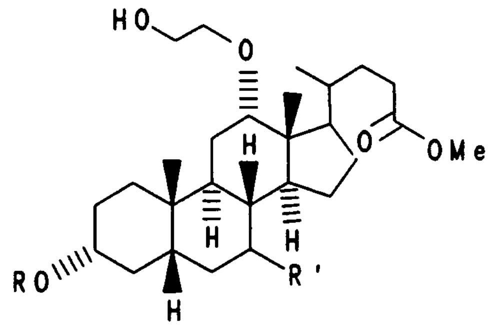

(X)

(XI)

R = H, Formyl, Acetyl, R' = H, OH, R'' = Formyl, Acetyl

VI

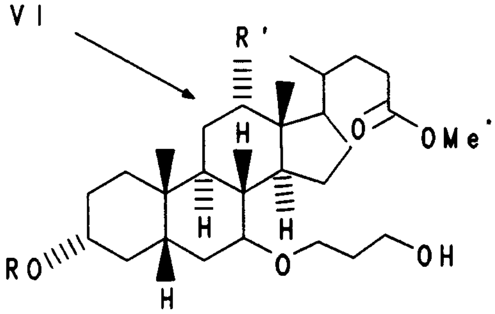

VII

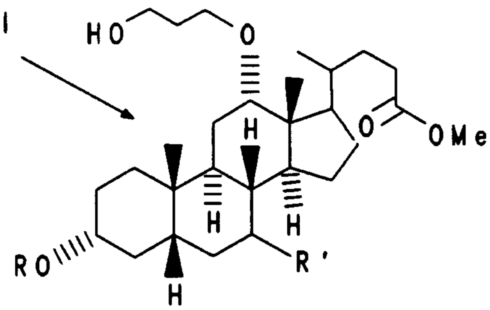

(XII)

(XIII)

X bzw. XII

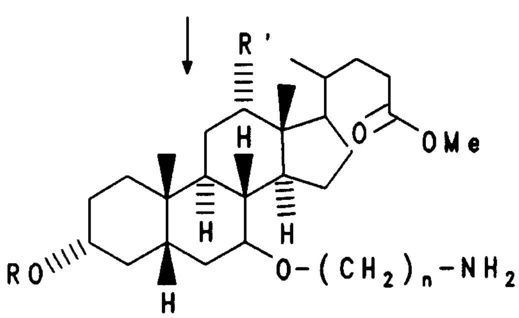

XI bzw. XIII

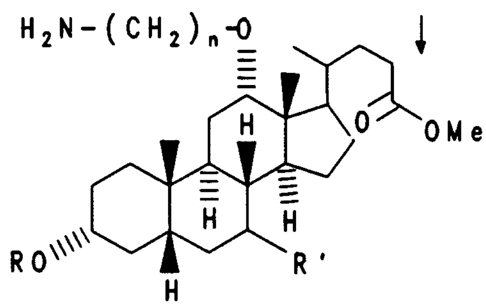

(XIV)

(XV)

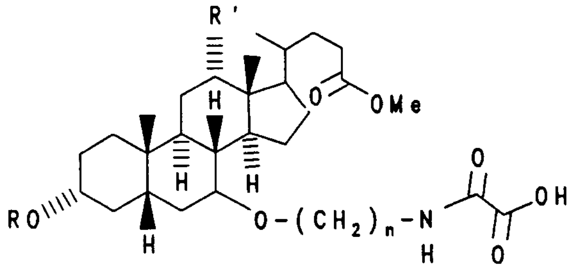

(XVI)

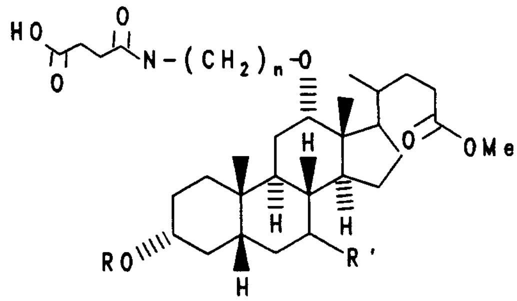

(XVII)

R = H, Formyl, Acetyl, R' = H, OH, n = 2,3

Verbindungen des Typs V, bei denen die 3-Position geschützt ist, werden mit Allylbromid/Hünigbase oder Triethylamin zur Reaktion gebracht. Besitzt die Verbindung V eine OH-Gruppe, ist die Alkylierung eindeutig, bei zwei freien OH-Gruppen wird zu etwa gleichen Teilen Monoalkylierung an den Positionen 7 und 12 erfolgen und nur Spuren des zweifach alkylierten Produkts entstehen. Die Schutzgruppe in 3-Position kann entweder mit Natriummethylat abgespalten oder aber auch für die weiteren Reaktionen beibehalten werden. Die monoalkylierten Verbindungen VI und VII können mit Ozon oder auch mit $OsO_4/NaJO_4$ zu den Aldehyden VIII und IX gespalten werden. Hieraus sind durch einfache Reduktion, z.B. mit $NaBH_4$, die 7- bzw. 12-Hydroxyethylverbindungen X und XI leicht zugänglich. Aus den Allylverbindungen VI und VII können durch Hydroborierung die entsprechenden 7- bzw. 12-Hydroxypropylderivate XII und XIII synthetisiert werden. Aus den Hydroxyalkylverbindungen des Typs X bis XIII können nach einer im Prinzip bekannten Reaktionsfolge (Mesylierung der primären OH-Gruppe mit Methansulfonsäurechlorid/Pyridin, Azidaustausch mit $NaN_3$ in Dimethylformamid, Reduktion der Azidfunktion mit Wasserstoff unter katalytischen Bedingungen) die Aminoalkylderivate XIV und XV hergestellt werden. Weitere Umsetzung der Aminofunktionen dieser Verbindungen mit Bernsteinsäureanhydrid liefert Gallensäurebausteine des Typs XVI und XVII. Außerdem sind in EP-A-0 489 423 geeignete Gallensäurebausteine beschrieben.

Die Erfindung betrifft weiterhin die Verwendung der erfindungsgemäßen Verbindungen zur Herstellung eines Heilmittels. Hierzu werden die Verbindungen der allgemeinen Formel I gelöst oder suspendiert in pharmakologisch unbedenklichen organischen Lösungsmitteln, wie ein- oder mehrwertigen Alkoholen, wie z.B. Ethanol oder Glycerin, in Triacetin, Ölen wie z.B. Sonnenblumenöl, Lebertran, Ethern, wie z.B. Diethylenglykoldimethylether oder auch Polyethern, wie z.B. Polyethylenglykol, oder auch in Gegenwart anderer pharmakologisch unbedenklicher Polymerträger, wie z.B. Polyvinylpyrrolidon, oder anderen pharmazeutisch annehmbaren Zusatzstoffen wie Stärke, Cyclodextrin oder Polysacchariden. Ferner können die erfindungsgemäßen Verbindungen in Kombination mit anderen Arzneistoffen gegeben werden.

Die Verbindungen der Formel I werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 5000 mg, vorzugsweise jedoch im Dosisbereich 10 bis 1000 mg.

Die Verbindungen eignen sich aufgrund ihrer pharmakologische Eigenschaften insbesondere als Hypolipidämika.

Die Erfindung betrifft daher auch Arzneimittel auf Basis der Verbindungen der Formel (I) sowie die Verwendung der Verbindungen als Arzneimittel, insbesondere zur Senkung des Cholesterinspiegels.

Die biologische Prüfung der erfindungsgemäßen Verbindungen erfolgte durch Ermittlung der Hemmung der [$^3$H]-Taurocholataufnahme in Bürstensaummembranvesikel des Ileums von Kaninchen. Der Hemmtest wurde wie folgt durchgeführt:

1. Präparation von Bürstensaummembranvesikeln aus dem Ileum von Kaninchen

Die Präparation von Bürstensaummembranvisikeln aus den Darmzellen des Dünndarm erfolgte mit der sogenannten $Mg^{2+}$-Präzipitationsmethode. Männliche Neuseeland-Kaninchen (2 bis 2,5 kg Körpergewicht) wurden durch intravenöse Injektion von 0,5 ml einer wäßrigen Lösung von 2,5 mg Tetracain HCl, 100 T 61$^R$ und 25 mg Mebezoniumjodid getötet. Der Dünndarm wurde entnommen und mit eiskalter physiologischer Kochsalzlösung gespült. Die terminalen 7/10 des Dünndarms (gemessen in oral rectaler Richtung, d. h. das terminale Ileum, welches das aktive $Na^+$-abhängige Gallensäuretransportsystem enthält) wurden zur Präparation der Bürstensaummembranvesikel verwendet. Die Därme wurden in Kunststoffbeuteln unter Stickstoff bei -80 °C eingefroren. Zur Präparation der Membranvesikel wurden die eingefrorenen Därme bei 30 °C im Wasserbad aufgetaut. Die Mucosa wurde abgeschabt und in 60 ml eiskaltem 12 mM Tris/HCl-Puffer (pH 7,1)/300 mM Mannit, 5 mM EGTA/10 mg/l Phenylmethylsulfonylfluorid/1 mg/l Trypsin Inhibitor v. Sojabohnen (32 U/mg)/0,5 mg/l Trypsin Inhibitor v. Rinderlunge (193 U/mg)15 mg/l Bacitracin suspendiert. Nach dem Verdünnen auf 300 ml mit eiskaltem destilliertem Wasser wurde mit einem Ultraturrax (18-Stab, IKA Werk Staufen, BRD) 3 Minuten bei 75 % max. Leistung unter Eiskühlung homogenisiert. Nach Zugabe von 3 ml 1 M $MgCl_2$-Lösung (Endkonzentration 10 mM) ließ man exakt 1 Minute bei 0 °C stehen. Durch Zugabe von $Mg^{2+}$ aggregieren die Zellmembranen und präzipitieren mit Ausnahme der Bürstensaummembranen. Nach einer 15-minütigen Zentrifugation bei 3000 x g (5000 rpm, SS-34-Rotor) wurde der Niederschlag verworfen und der Überstand, der die Bürstensaummembranen enthält, 30 Minuten bei 267000 x g (15000 rpm, SS-34-Rotor) zentrifugiert. Der Überstand wurde verworfen, der Niederschlag in 60 ml 12 mM Tris/HCl-Puffer (pH 7,1)/60 mM Mannit, 5 mM EGTA mit einem Potter Elvejhem Homogenisator (Braun, Melsungen, 900 rpm, 10 Hübe) rehomogenisiert. Nach Zugabe von 0,1 ml 1 M $MgCl_2$-Lösung und 15-minütiger Inkubationszeit bei 0 °C wurde erneut 15 Minuten bei 3000 x g zentrifugiert. Der Überstand wurde anschließend nochmals 30 Minuten bei 46000 x g (15000 rpm, SS-34-Rotor) zentrifugiert. Der Niederschlag wurde in 30 ml 10 mM Tris/Hepes-Puffer (pH 7,4)/300 mM Mannit aufgenommen und durch 20 Hübe in einem Potter Elvejhem Homogenisator bei 1000 rpm homogen resuspendiert. Nach 30 minütiger Zentrifugation bei 48000 x g (20000 rpm, SS-34-Rotor) wurde der Niederschlag in 0,5 bis 2 ml Tris/Hepes-Puffer (pH 7,4)/280 mM Mannit (Endkonzentration 20 mg/ml) aufgenommen und mit Hilfe einer Tuberkulinspritze mit einer 27 Gauge-Nadel resuspendiert. Die Vesikel wurden entweder unmittelbar nach der Präparation für Transportuntersuchungen verwendet oder bei -196 °C in 4 mg Portionen in flüssigem Stickstoff aufbewahrt.

2. Hemmung der $Na^+$-abhängigen [$^3$H]Taurocholat-Aufnahme in Bürstensaummembranvesikel des Ileums

Die Aufnahme von Substraten in die vorstehend beschriebenen Bürstensaummembranvesikel wurde mittels der sogenannten Membranfiltrationstechnik bestimmt. 10 µl der Vesikelsuspension (100 µg Protein) wurden als Tropfen an die Wand eines Polystyrolinkubationsröhrchens (11 x 70 mm) pipettiert, welches das Inkubationsmedium mit den entsprechenden Liganden enthielt (90 µl). Das Inkubationsmedium enthielt 0,75 µl = 0,75 µCi [$^3$H(G)]-Taurocholat (spezifische Aktivität: 2,1 Ci/mMol) /0,5 µl 10 mM Taurocholat/8,75 µl Natrium-Transport-Puffer (10 mM Tris/Hepes, (pH 7,4)/100 mM Mannit/100 mM NaCl) (Na-T-P) bzw. 8,75 µl Kalium-Transport-Puffer (10 mM Tris/Hepes (pH 7,4)/100 mM Mannit/100 mM KCl) (K-T-P) und 80 µl der betreffenden Inhibitorlösung, je nach Experiment in Na-T-Puffer bzw. K-T-Puffer gelöst. Das Inkubationsmedium wurde durch ein Polyvinylidenfluorid-Membranfilter (SYHV LO 4NS, 0,45 µm, 4 mm Ø, Millipore, Eschborn, BRD) filtriert. Durch Vermischung der Vesikel mit dem Inkubationsmedium wurde die Transportmessung gestartet. Die Konzentration an Taurocholat im Inkubationsansatz betrug 50 µM. Nach der gewünschten Inkubationszeit (üblicherweise 1 Minute) wurde der Transport durch Zugabe von 1 ml eiskalter Stoplösung (10 mM Tris/Hepes, (pH 7,4)/150 mM KCl) gestoppt.

Die entstehende Mischung wurde sofort bei einem Vakuum von 25 bis 35 mbar über ein Membranfilter aus Cellulosenitrat (ME 25, 0,45 µm, 25 mm Durchmesser, Schleicher & Schuell, Dassell, BRD) abgesaugt. Der Filter wurde mit 5 ml eiskalter Stoplösung nachgewaschen.

Zur Messung der Aufnahme des radioaktiv markierten Taurocholats wurde das Membranfilter mit 4 ml des Szintillators Quickszint 361 (Zinsser Analytik GmbH, Frankfurt, BRD) aufgelöst und die Radioaktivität durch Flüssigkeits-Szintillationsmessung in einem Meßgerät TriCarb 2500 (Canberra Packard GmbH, Frankfurt, BRD) gemessen. Die gemessenen Werte wurden nach Eichung des Gerätes mit Hilfe von Standardproben und nach Korrektur evtl. vorhandener Chemilumineszenz als dpm (Decompositions per Minute) erhalten.

Die Kontrollwerte wurden jeweils in Na-T-P und K-T-P ermittelt. Die Differenz zwischen der Aufnahme in Na-T-P und K-T-P ergab den $Na^+$-abhängigen Transportanteil. Als $IC_{50}$ $Na^+$ wurde diejenige Konzentration an Inhibitor bezeichnet, bei der der $Na^+$-abhängige Transportanteil um 50 % - bezogen auf die Kontrolle - gehemmt war.

Die pharmakologischen Daten umfassen eine Testserie, in der die Interaktion der erfindungsgemäßen Verbindungen mit dem intestinalen Gallensäuretransportsystem im terminalen Dünndarm untersucht wurde. Die Ergebnisse sind in Tabelle 11 zusammengefaßt.

Beispiel 1 und 2

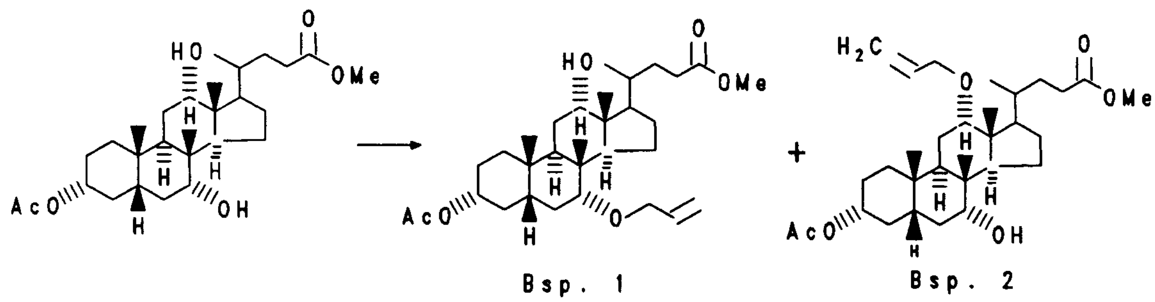

150 g (0,32 mol) 3-Acetyl-cholsäuremethylester, 500 ml Dimethylformamid, 125 ml N-Ethyl-diisopropylamin und 70 ml Allylbromid werden 16 Stunden unter Rückfluß erhitzt. Jeweils nach 2 Stunden wird neues Allylbromid (25 ml) zugegeben. Die Reaktionslösung wird einrotiert. Der Rückstand wird zwischen Wasser/Methylenchlorid verteilt, die organische Phase abgetrennt und mit Magnesiumsulfat getrocknet. Nach Säulenchromatographie (Ethylacetat/Cyclohexan 1:2, Kieselgel 70-200) µm werden die Produktfraktionen einrotiert.
Ausbeute = 92,2 g 7-/12-Allyl-Gemisch.
$C_{30}H_{48}O_6$ (504), MS 511 ($M + Li^+$)
Das Gemisch wurde durch fraktionierte Kristallisation mit n-Heptan getrennt.

Beispiel 3

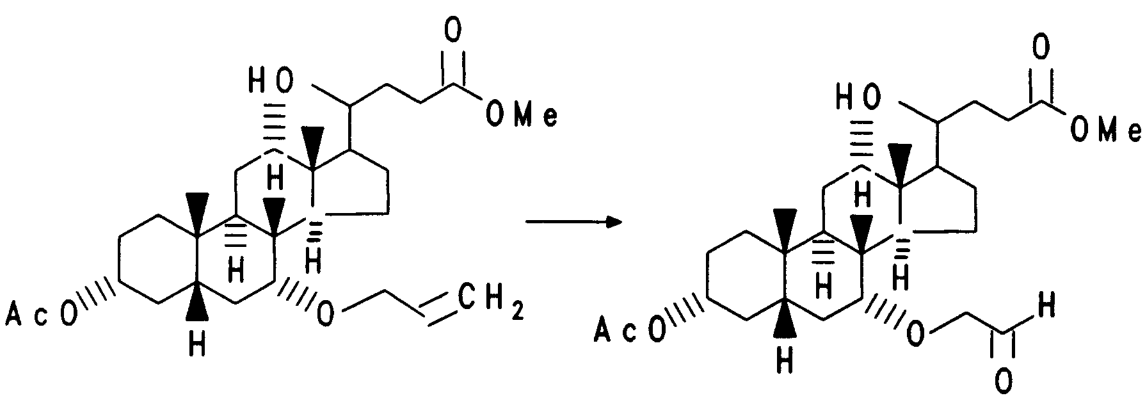

50 g (0,1 mol) Bsp. 1, 250 ml Diethylether und 250 ml Wasser werden unter starkem Rühren vorgelegt. Es werden 503 mg (0,002 mol) Osmiumtetroxid zugegeben. Es wird 15 Min. bei Raumtemperatur gerührt. 53 g (0,25 mol) Natriumperjodat werden über 1 Std. portionsweise zugegeben, und es wird 8 Stunden unter starkem Rühren nachgerührt. Die Etherphase wird abgetrennt, mit Magnesiumsulfat getrocknet und einrotiert.
Ausbeute: 47 g Roh $C_{29}H_{46}O_7$ (506), MS 513 ($M + Li^+$)
Beispiel 3 wird ohne weitere Reinigung weiter umgesetzt.

Beispiel 4

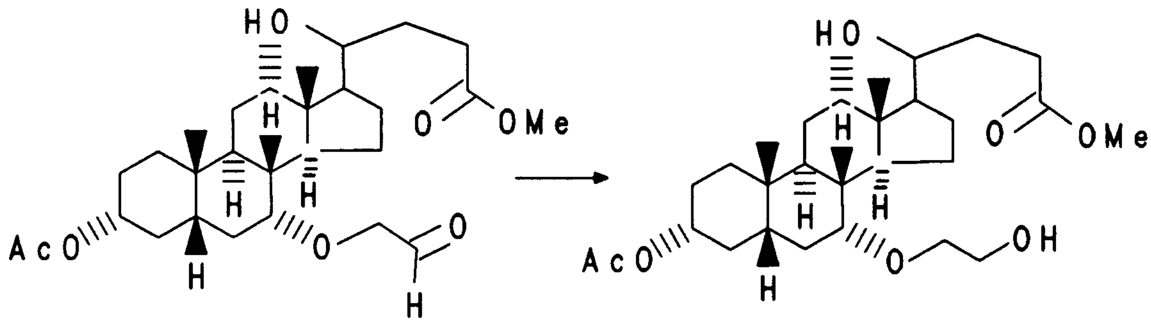

Zu 47 g (0,093 mol) Bsp. 3 und 250 ml Methanol werden bei 0 °C, 4,2 g (0,11 mol) Natriumborhydrid portionsweise zugegeben. Nach 2 Stunden bei 0 °C wird die Reaktionslösung auf gesättigte Ammoniumchlorid-Lösung gegossen, 3mal mit Essigester extrahiert und die vereinten organischen Phasen werden mit Magnesiumsulfat getrocknet und einrotiert. Nach Säulenchromatographie (Ethylacetat/Cyclohexan 1,5:1, Kieselgel 35 - 70 µm) werden die Produktfraktionen einrotiert und mit Diisopropylether kristallisiert.
Ausbeute: 25 g $C_{29}H_{48}O_7$ (508), MS 515 ($M+Li^+$)

Beispiel 5

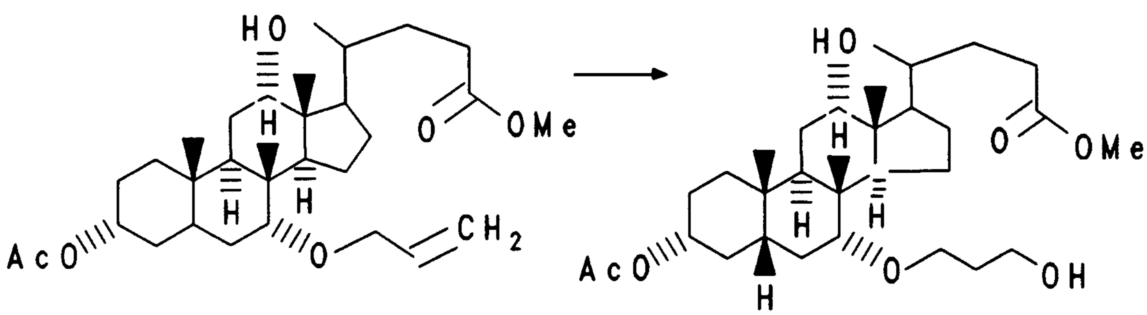

10 g (0,02 mol) Bsp. 1 und 250 ml Tetrahydrofuran wurden bei Raumtemperatur vorgelegt und 40 ml (0,04 mol) Boran-Tetrahydrofuran-Komplex (1 molar) bei Raumtemperatur zugetropft. Es wurde 2 Stunden bei Raumtemperatur nachgerührt und nacheinander wurden 25 ml Wasser, 25 ml 2-n-Natronlauge und 25 ml 35 %ige Wasserstoffperoxidlösung zugetropft. Es wurde weitere 15 Min. bei Raumtemperatur nachgerührt. Die Reaktionslösung wurde auf Wasser gegossen, 3 x mit Diethylether extrahiert, die vereinten organischen Phasen wurden mit Magnesiumsulfat getrocknet und einrotiert.
Ausbeute: 8,5 g $C_{30}H_{50}O_7$ (522), MS 529 ($M+Li^+$)
Beispiel 5 wurde ohne weitere Reinigung weiter umgesetzt.

Beispiel 6

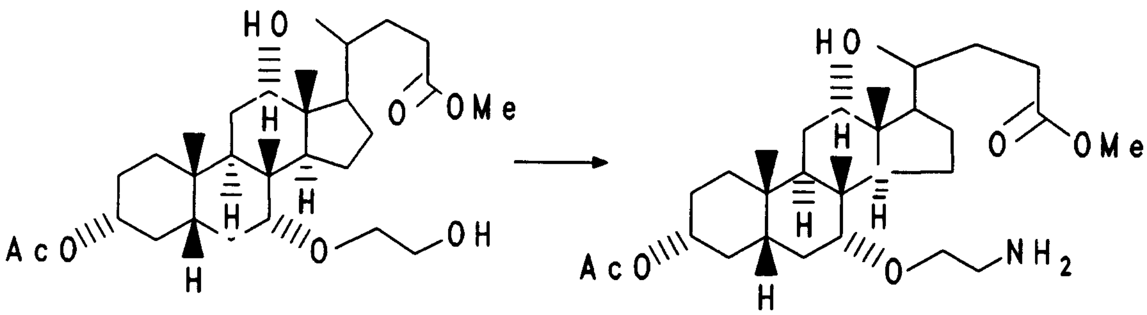

10 g (0,02 mol) Bsp. 4 und 100 ml Pyridin werden bei 0 ° C vorgelegt. Es werden 1,7 ml (0,022 mol) Methansulfonsäurechlorid bei 0 ° C zugetropft und es wird noch 30 Min bei 0 ° C und 2 Std. bei Raumtemperatur nachgerührt. Die Reaktionslösung wird auf Wasser gegossen und 3x mit Essigester extrahiert, die vereinten organischen Phasen werden mit Magnesiumsulfat getrocknet und einrotiert. Der Rückstand wird in 100 ml Dimethylformamid gelöst, mit 1,4 g (0,022 mol) Natriumazid versetzt und 2 Std. bei 80 ° C gerührt. Die Reaktionslösung wird auf Wasser gegossen und wie oben beschrieben aufgearbeitet. Der Rückstand wird in 100 ml Methanol gelöst, mit 100 mg Palladium auf Kohle (10 %ig) versetzt und 2 Std. bei Normaldruck hydriert. Es wird vom Katalysator abfiltriert und das Filtrat wird einrotiert. Nach Säulenchromatographie (Ethylacetat/MeOH/$Et_3N$ 10:1:1, Kieselgel 70-200 µm) erhält man Beispiel 6.
Ausbeute = 7,3 g $C_{29}H_{49}NO_6$ (507), MS 514 ($M + Li^+$)

Beispiel 7

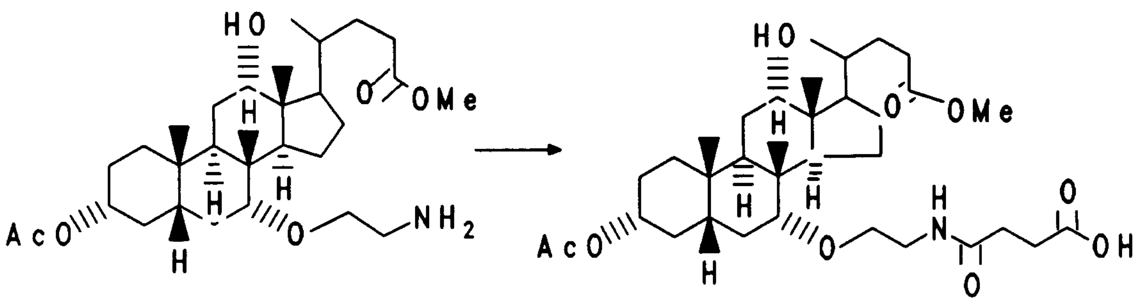

Zu 500 mg (0,001 mol) Aminoverbindung, 20 ml Tetrahydrofuran und 4 ml Triethylamin werden bei Raumtemperatur, 98,6 mg (0,001 mol) Bernsteinsäureanhydrid zugegeben. Es wird 1 Std. bei Raumtemperatur nachgerührt. Die Reaktionslösung wird auf 25 %ige Natriumdihydrogenphosphatlösung gegossen, 3 x mit Essigester extrahiert, die organische Phase mit Magnesiumsulfat getrocknet und einrotiert.
Ausbeute: 580 mg $C_{33}H_{53}NO_9$ (607), MS 614 ($M + Li^+$)
Beispiel 7 wurde ohne weitere Reinigung weiter umgestzt.

Analog zu den Beispielen 3 bis 7 wurden die Beispiele 8 bis 12 hergestellt.

Beispiele 8-12

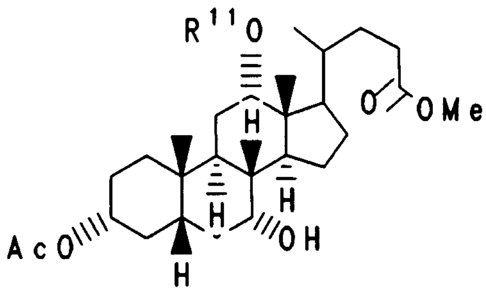

| Beispiel | $R^{11}$ | MS |
|---|---|---|
| 8 | $-CH_2CHO$ | 513 ($M+Li^+$) |
| 9 | $-CH_2CH_2OH$ | 515 ($M+Li^+$) |
| 10 | $-CH_2CH_2CH_2OH$ | 529 ($M+Li^+$) |
| 11 | $-CH_2CH_2NH_2$ | 514 ($M+Li^+$) |
| 12 | $-CH_2CH_2NHCOCH_2CH_2COOH$ | 614 ($M+Li^+$) |

Beispiel 13

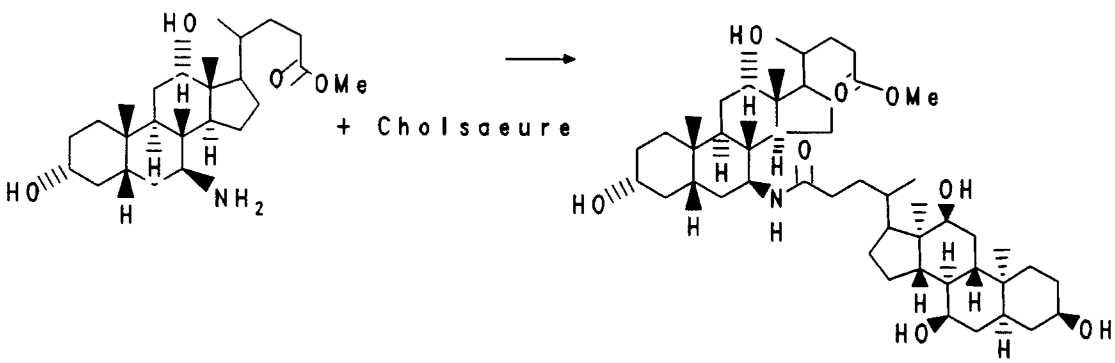


300 mg (0,73 mmol) Cholsäure, 330 mg (0,78 mmol) 7$\beta$-Amino-3$\alpha$,12$\alpha$-dihydroxy-5$\beta$-cholansäuremethylester (Redel, Bull. Soc. Chim. Fr., S. 877, 1949), 240 mg (0,97 mmol) EEDQ und 0,25 ml Diisopropylethylamin werden in 20 ml DMF 4 Stunden bei 90 °C gerührt. Nach dem Abkühlen wird das Reaktionsgemisch eingeengt und der Rückstand über Kieselgel chromatographiert ($CH_2Cl_2$/MeOH 8.2).$C_{49}H_{81}NO_8$ (812) 819 ($M+Li^+$). Die Verknüpfung der beiden Gallensäurederivate kann auch mit Triethylamin in Dichlormethan oder mit Dicyclohexylcarbodiimid, Hydroxybenzotriazol oder Triethylamin in Tetrahydrofuran durchgeführt werden.

Analog zu Beispiel 13 wurden die Verbindungen der Tabelle 1 hergestellt.

Tabelle 1

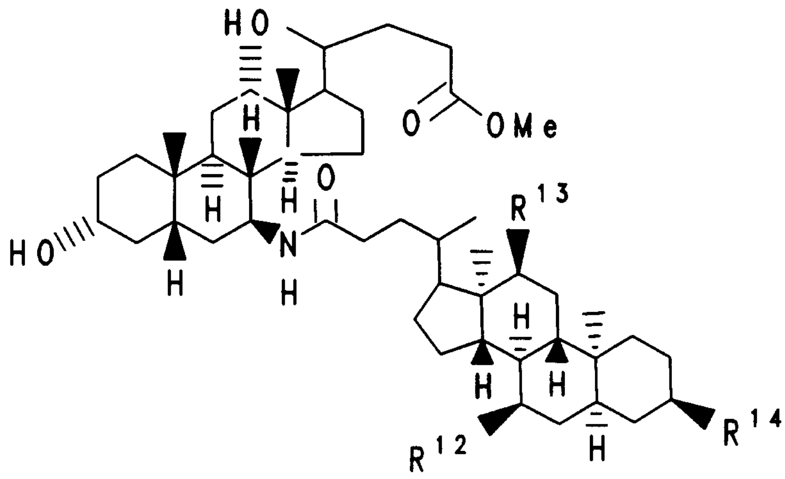

| Beispiel | $R^{12}$ | $R^{13}$ | $R^{14}$ | MS (FAB, 3-NBA/LiCl) |
|---|---|---|---|---|
| 14 | α-OH | H | -OH | $C_{49}H_{81}NO_7$ (796) 803 ($M+Li^+$) |
| 15 | ß-OH | H | -OH | $C_{49}H_{81}NO_7$ (796) 803 ($M+Li^+$) |
| 16 | H | H | -OCHO | $C_{50}H_{81}NO_7$ (808.5) 809.5 ($M+H^+$) |

Analog Zu Beispiel 13 wurden aus den Beispielen 7 und 8 die Beispiele der Tabelle 2 erhalten.

Tabelle 2

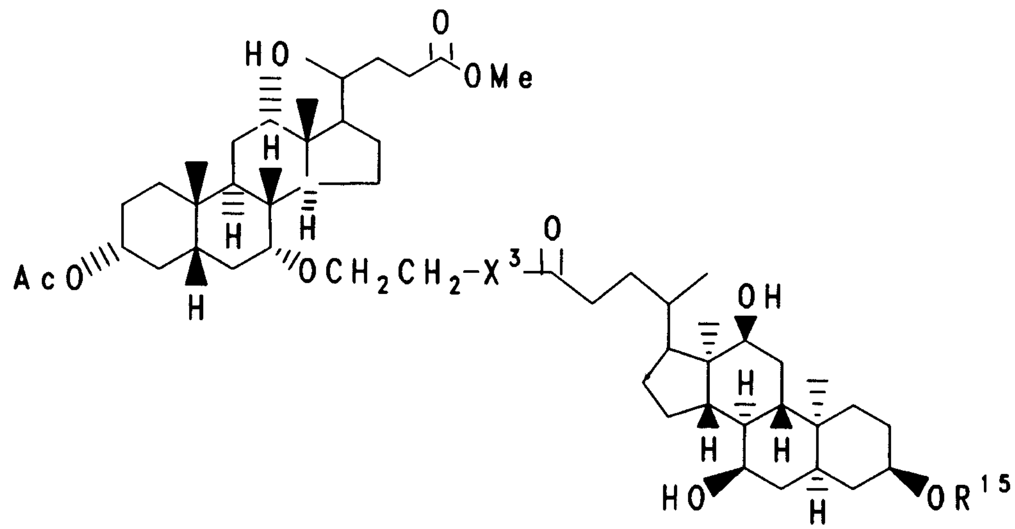

| Beispiel | $X^3$ | $R^{15}$ | MS (FAB, 3-NBA/LiCl) |
|---|---|---|---|
| 17 | -NH- | H | $C_{53}H_{87}NO_{10}$ (898) 905 ($M+Li^+$) |
| 18 | -NH- | Diphenylmethyl | $C_{66}H_{97}NO_{10}$ (1064) 1071 ($M+Li^+$) |
| 19 | $-NHCO(CH_2)_2CONH(CH_2)_3NH-$ | H | $C_{60}H_{99}N_3O_{12}$ (1054) 1061 ($M+Li^+$) |
| 20 | $-NHCO(CH_2)_2CONH(CH_2)_3NH-$ | Diphenylmethyl | $C_{73}H_{109}N_3O_{12}$ (1220) 1227 ($M+Li^+$) |

Ebenfalls analog Zu Beispiel 13 wurden die Beispiele der Tabellen 3 und 4 erhalten.

Tabelle 3

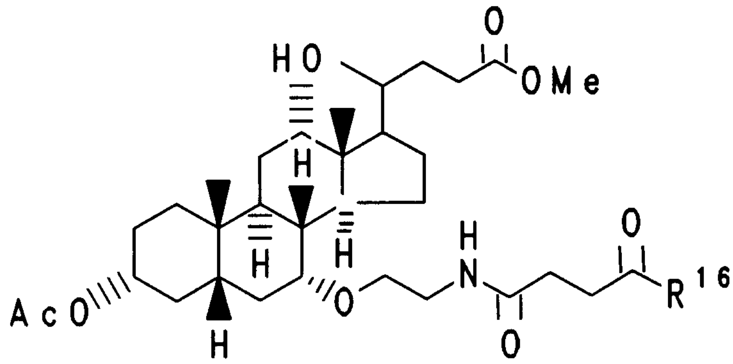

| Beispiel | $R^{16}$ | MS (FAB, 3-NBA/LiCl) |
|---|---|---|
| 21 | 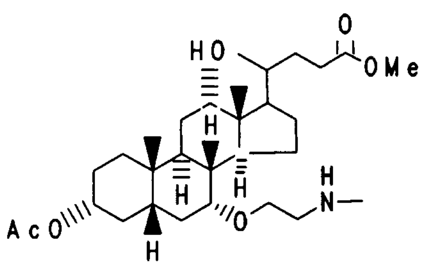 | $C_{62}H_{100}N_2O_{14}$ (1097) 1104 ($M+Li^+$) |
| 22 | 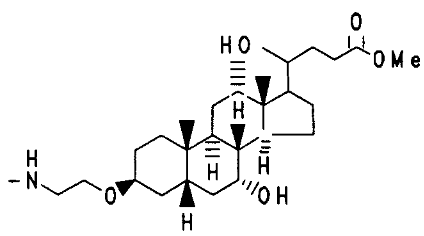 | $C_{60}H_{98}N_2O_{13}$ (1055) 1062 ($M+Li^+$) |

Tabelle 4

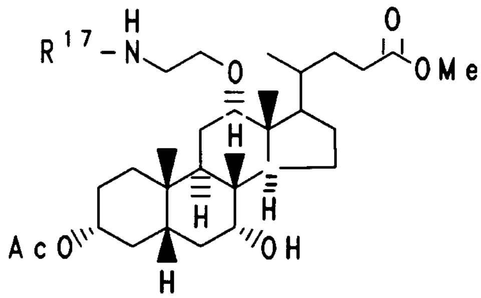

| Beispiel | $R^{17}$ | MS (FAB, 3-NBA/LiCl) |
|---|---|---|
| 23 | 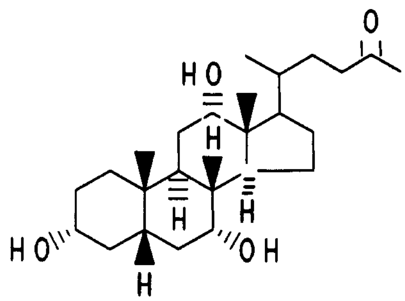 | $C_{53}H_{87}NO_{10}$ (898) 905 ($M+Li^+$) |
| 24 | 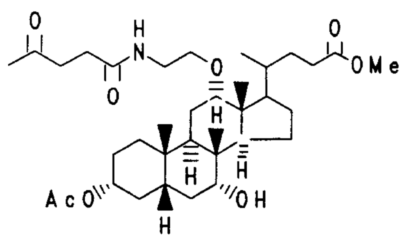 | $C_{62}H_{100}N_2O_{14}$ (1097) 1104 ($M+Li^+$) |

| Beispiel | $R^{17}$ | MS (FAB, 3-NBA/LiCl) |
|---|---|---|
| 25 | 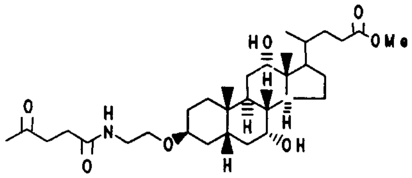 | $C_{60}H_{98}NO_{13}$ (1055) 1062 ($M+Li^+$) |

Beispiel 26

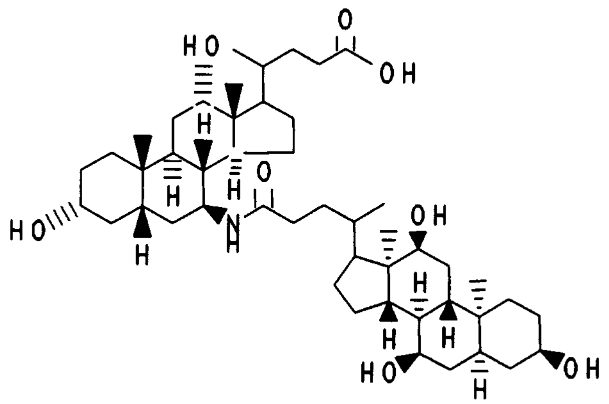

250 mg (0,31 mmol) Beispiel 13 werden in 20 ml Ethanol gelöst, mit 2 ml 1 N-NaOH-Lösung versetzt und 16 h bei Zimmertemperatur gerührt. Zur Aufarbeitung wird eingeengt, in $H_2O$ gelöst, mit 2 N HCl auf pH 1-2 gebracht und erneut eingeengt. Der Rückstand wird über Kieselgel chromatographiert ($CHCl_3$/MeOH 8:2). Es werden 220 mg freie Säure erhalten (90 %).
MS (FAB, 3-NBA/LiCl) $C_{48}H_{79}NO_8$ (798) 805 ($M+Li^+$)
Analog zu Beispiel 26 werden die Beispiele der Tabellen 5 bis 8 erhalten.

Tabelle 5

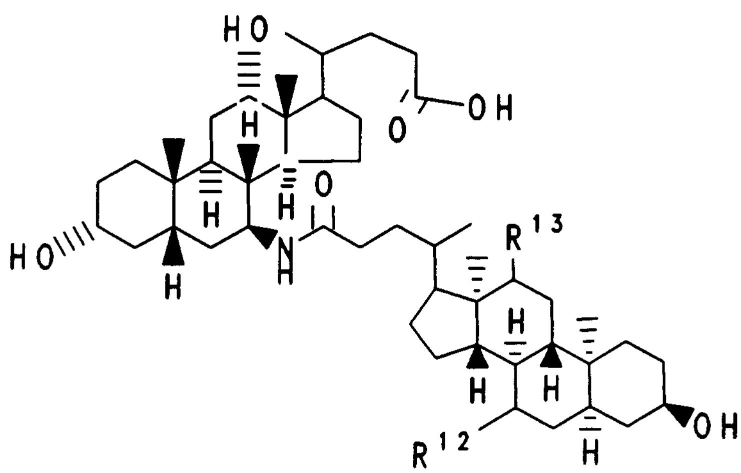

| Beispiel | $R^{12}$ | $R^{13}$ | MS |
|---|---|---|---|
| 27 | α-OH | H | $C_{48}H_{79}NO_7$ (782) 789 ($M+Li^+$) |
| 28 | ß-OH | H | $C_{48}H_{79}NO_7$ (782) 789 ($M+Li^+$) |
| 29 | H | H | $C_{48}H_{79}NO_7$ (766) 773 ($M+Li^+$) |

Tabelle 6

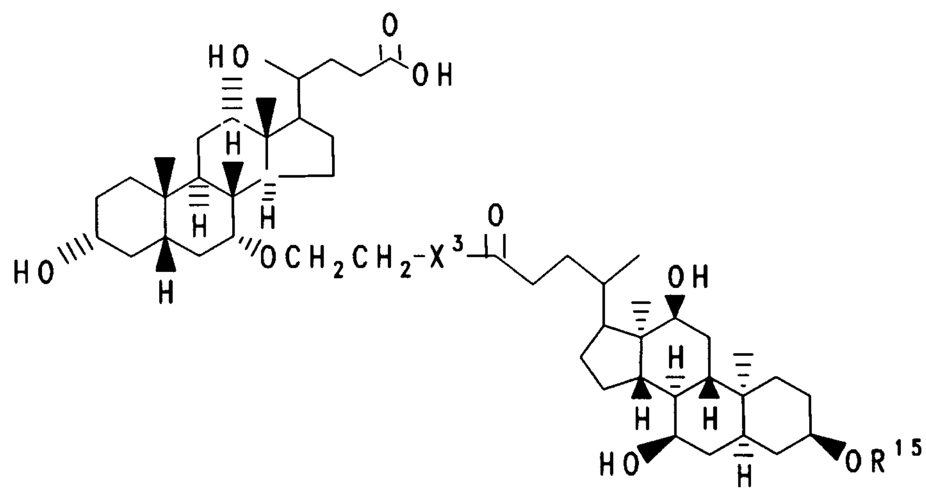

| Beispiel | $X^3$ | $R^{15}$ | MS |
|---|---|---|---|
| 30 | -NH- | H | $C_{50}H_{85}NO_{10}$ (860) 867 ($M+Li^+$) |
| 31 | -NH- | Diphenylmethyl | $C_{63}H_{95}NO_{10}$ (1026) 1033 ($M+Li^+$) |
| 32 | $-NHCO(CH_2)_2CONH(CH_2)_3NH-$ | H | $C_{57}H_{97}N_3O_{12}$ (1016) 1023 ($M+Li^+$) |
| 33 | $-NHCO(CH_2)_2CONH(CH_2)_3NH-$ | Diphenylmethyl | $C_{70}H_{107}N_3O_{12}$ (1182) 1189 ($M+Li^+$) |

Tabelle 7

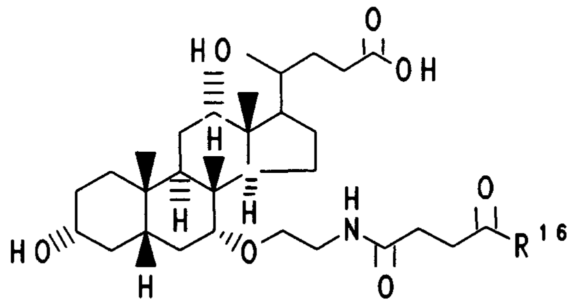

| Beispiel | $R^{16}$ | MS |
|---|---|---|
| 34 | 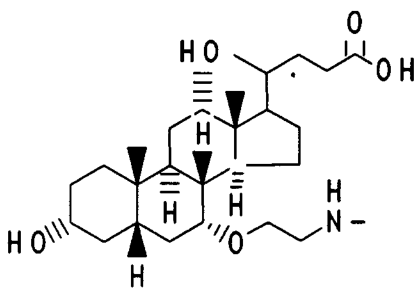 | $C_{56}H_{92}N_2O_{12}$ (985) 992 (M+Li$^+$) |
| 35 | 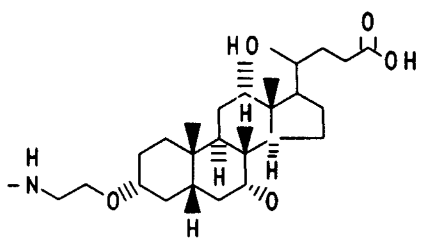 | $C_{58}H_{96}N_2O_{12}$ (985) 992 (M+Li$^+$) |

Tabelle 8

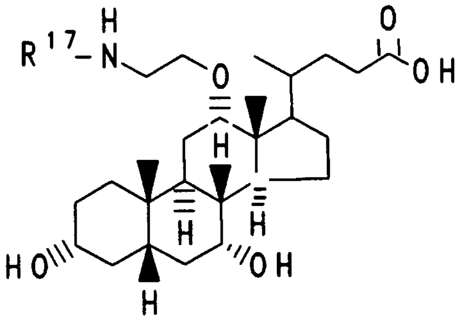

| Beispiel | $R^{17}$ | MS (FAB, 3-NBA/LiCl) |
|---|---|---|
| 36 | 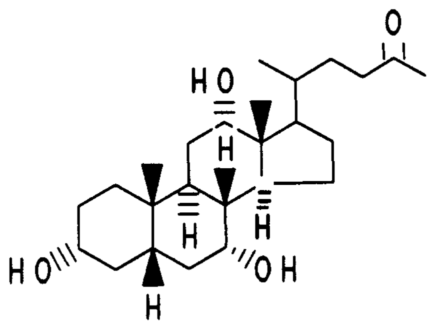 | $C_{50}H_{83}NO_9$ (842) 849 (M+Li$^+$) |
| 37 | 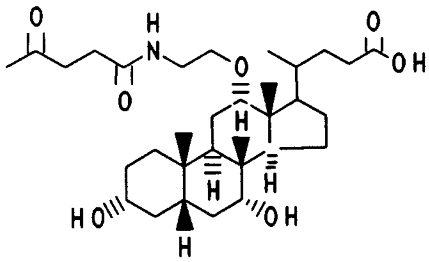 | $C_{56}H_{92}N_2O_{12}$ (985) 992 (M+Li$^+$) |

| Beispiel | $R^{17}$ | MS (FAB, 3-NBA/LiCl) |
|---|---|---|
| 38 | 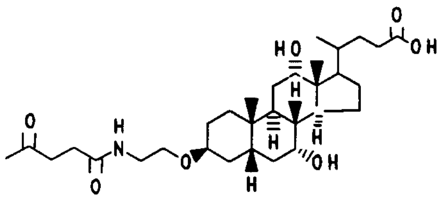 | $C_{56}H_{92}N_2O_{12}$ (985) 992 ($M+Li^+$) |

Analog zu bereits beschriebenen Syntheseverfahren (EP 489 423) wurden die folgenden Glycin- und Taurinkonjugate erhalten.

Tabelle 9

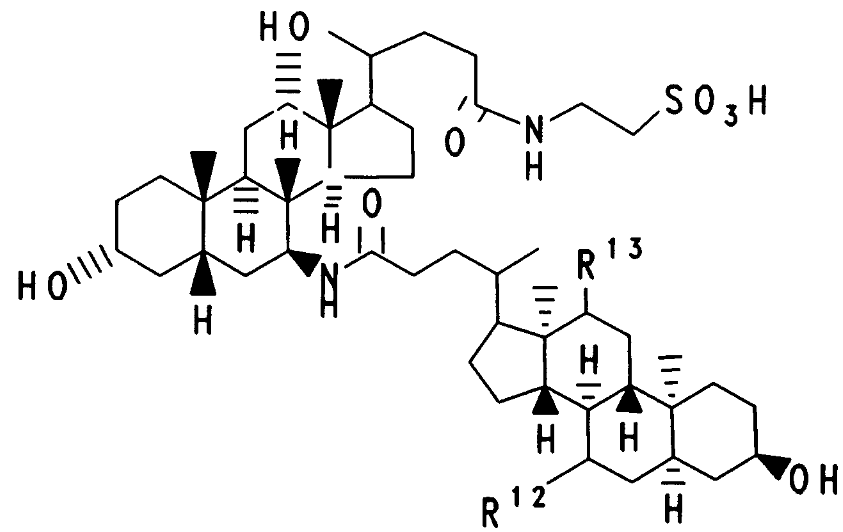

| Beispiel | $R^{12}$ | $R^{13}$ | MS (FAB, 3-NBA/LiCl |
|---|---|---|---|
| 39 | α-OH | α-OH | $C_{50}H_{84}N_2O_{10}S$ (905) 918 ($M+2Li^+-H^+$) |
| 40 | α-OH | H | $C_{50}H_{84}N_2O_9S$ (889) 890 ($M+H^+$) |
| 41 | ß-OH | H | $C_{50}H_{84}N_2O_9S$ (889) 912 ($M+Na^+$) |
| 42 | H | H | $C_{50}H_{84}N_2O_8S$ (872.5) 895.5 ($M+Na^+$) |

Tabelle 10

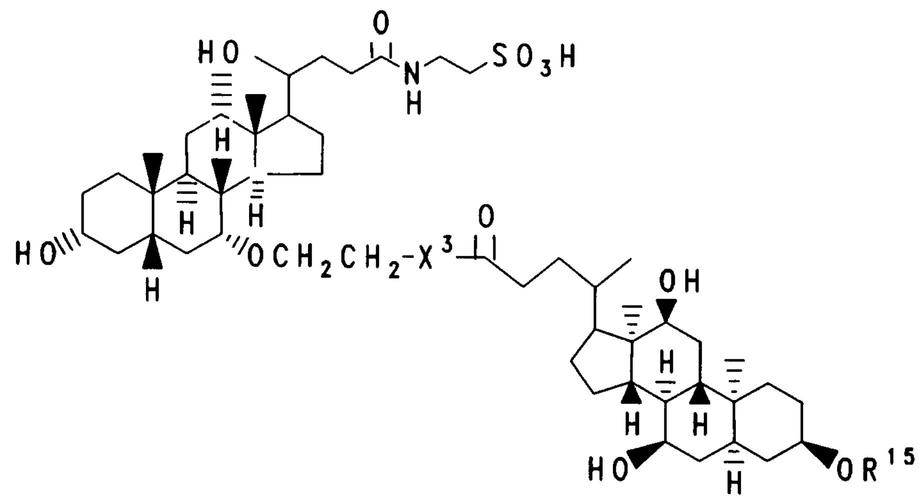

| Beispiel | $X^3$ | $R^{15}$ | MS |
|---|---|---|---|
| 43 | -NH- | H | $C_{52}H_{90}N_2O_{12}S$ (967) 974 ($M+Li^+$) |
| 44 | -NH- | Diphenylmethyl | $C_{65}H_{100}N_2O_{12}S$ (1133) 1140 ($M+Li^+$) |
| 45 | $-NHCO(CH_2)_2CONH(CH_2)_3NH-$ | H | $C_{55}H_{102}N_4O_{14}S$ (1123) 1130 ($M+Li^+$) |
| 46 | $-NHCO(CH_2)_2CONH(CH_2)_3NH-$ | Diphenylmethyl | $C_{72}H_{102}N_4O_{14}S$ (1289) 1296 ($M+Li^+$) |

Beispiel 47

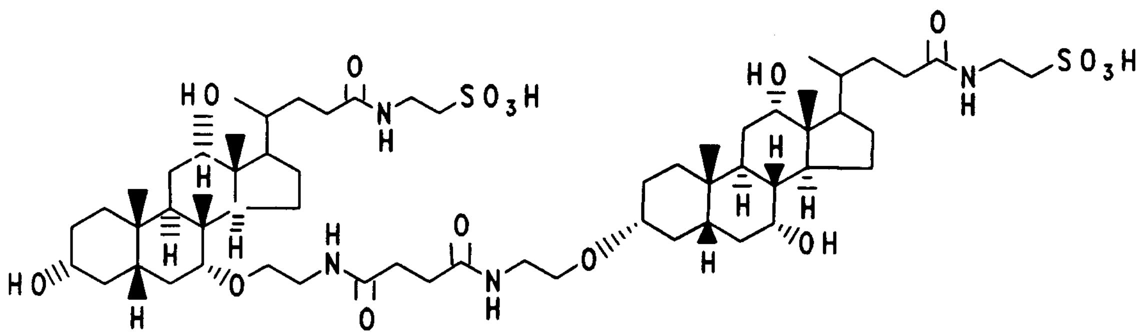

MS (FAB, 3-NBA/LiCl) $C_{60}H_{101}N_3O_{14}S$ (1092) 1099 (M + $Li^+$)

Beispiel 48

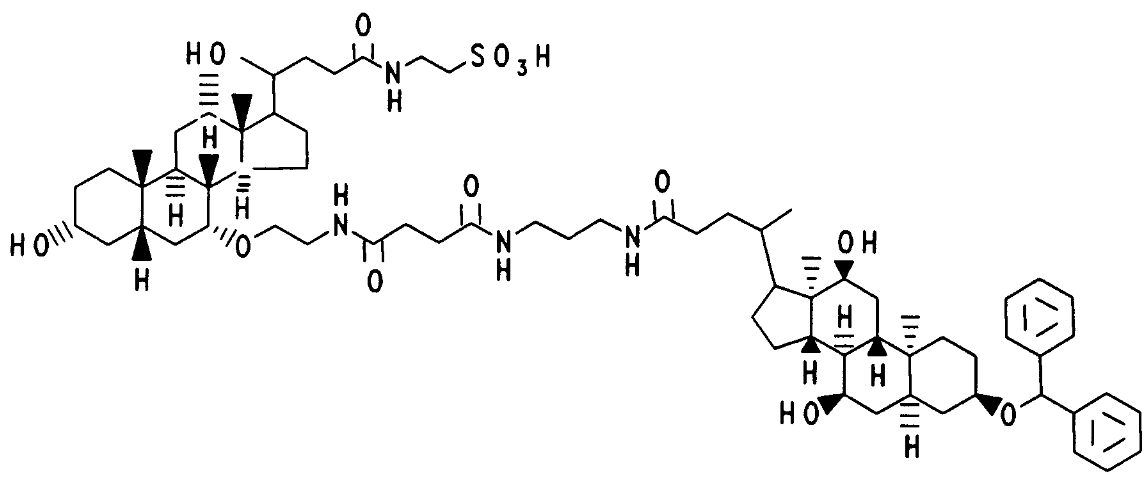

MS (FAB, 3-NBA/LiCl) $C_{72}H_{110}N_4O_{13}$ (1239) 1246 (M + $Li^+$)

Tabelle 11 zeigt Meßwerte der Hemmung der [$^3$H]-Taurocholataufnahme in Bürstensaummembranvesikel des Ileums von Kaninchen. Angegeben sind die Quotienten aus den $IC_{50}$- bzw. $IC_{50Na}$-Werten der Referenzsubstanz Taurochenodesoxycholat (TCDC) und der jeweiligen Testsubstanz.

Tabelle 11

| Verbindung aus Beispiel | $\frac{IC_{50}\text{-}TCDC[\mu mol]}{IC_{50}\text{-}Substanz[\mu mol]}$ | $\frac{IC_{50_{Na}}\text{-}TCDC[\mu mol]}{IC_{50_{Na}}\text{-}Substanz[\mu mol]}$ |
|---|---|---|
| 20 | 0,00 | 0,12 |
| 26 | 0,00 | 0,29 |
| 27 | 0,64 | 0,44 |
| 28 | 0,54 | 0,43 |
| 29 | 0,23 | 0,17 |
| 30 | 0,93 | 0,85 |
| 32 | 1,00 | 0,80 |
| 39 | 0,92 | 1,05 |
| 40 | 0,54 | 0,52 |
| 43 | 1,18 | 0,96 |
| 47 | 0,35 | 0,26 |
| 48 | 0,75 | 0,71 |

**Patentansprüche**

**1.** Gallensäurederivate der Formel I

G1 - X - G2 I

in der G1 einen Rest der Formel II bedeutet,

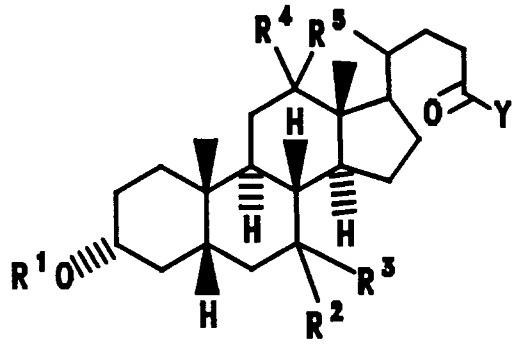

(II)

wobei

Y die folgende Bedeutung hat: OKa, wobei Ka Alkali-, Erdalkali- oder ein quartäres Ammoniumion bedeutet
-OL, -NHL, $-NL_2$,
eine über die Aminogruppe gebundene Aminosäure oder Aminosulfonsäure, wie z.B.
$-NHCH_2COOH$, $-NHCH_2CH_2SO_3H$,

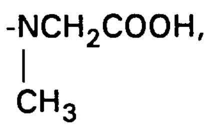 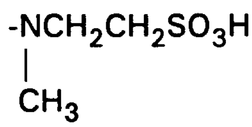

und deren $(C_1-C_4)$-Alkylester, Alkali- und Erdalkalisalze sowie quartäre Ammoniumsalze, und wobei L
H, einen Alkyl- oder Alkenylrest mit bis zu 10 C-Atomen, der verzweigt oder unverzweigt ist, einen Cycloalkylrest mit 3 bis 8 C-Atomen, einen Phenyl- oder Benzylrest, die unsubstituiert oder 1- bis 3-fach substituiert sind mit F, Cl, Br, $(C_1-C_4)$-Alkyl oder $(C_1-C_4)$-Alkoxy,

$R^1$ H, einen Alkyl- oder Alkenylrest mit bis zu 10 C-Atomen, der verzweigt oder unverzweigt ist, einen Cycloalkylrest mit 3 bis 8 C-Atomen,
einen Benzylrest,
einen Biphenylmethyl- oder einen Triphenylmethylrest,
bei denen die Kerne unsubstituiert oder 1 - bis 3-fach mit F, Cl, Br, $(C_1-C_4)$-Alkyl, $(C_1-C_4)$-Alkoxy substituiert sind,
einen Rest

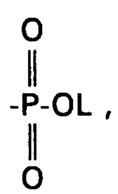 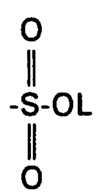 oder 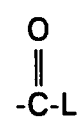

wobei L die oben angegebene Bedeutung hat,

$R^2$ bis $R^5$ wobei $R^2$ und $R^3$ bzw. $R^4$ und $R^5$ jeweils gemeinsam der Sauerstoff einer Carbonylgruppe oder einzeln und jeweils unabhängig voneinander
H, -OT, -ST, -NHT,

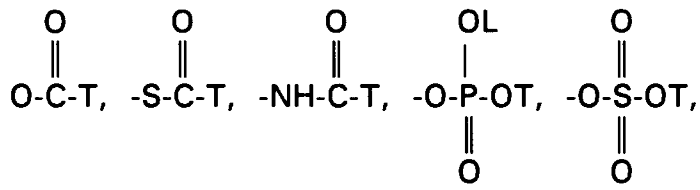

-T
wobei T die Bedeutung von L hat oder eine freie Valenz zur Bindung der Gruppe X aufweist,
wobei insgesamt von G1 nur eine freie Valenz zur Bindung der Gruppe X ausgeht,

X eine Einfachbindung oder eine Gruppe der Formel III ist

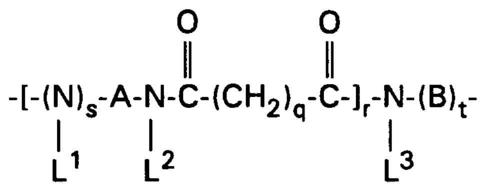

(III),

wobei

A und B Alkylenketten, die verzweigt oder unverzweigt sind, wobei die Ketten gegebenenfalls durch -O- oder -S- unterbrochen sein können,
$L^1$, $L^2$, $L^3$ gleich oder verschieden sind und die Bedeutung von L haben sowie

q null bis 5

r null oder 1

s null oder 1

t null oder 1 bedeuten

G2 einen Rest der Formel IV bedeutet

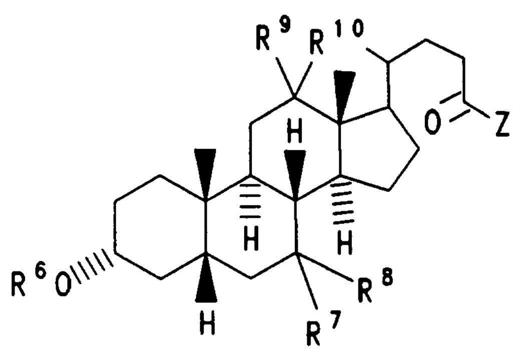

( I V )

wobei

Z eine freie Valenz zur Gruppe X ist oder die unter Y angegebene Bedeutung hat,

$R^6$ eine freie Valenz zur Gruppe X ist oder die unter $R^1$ angegebene Bedeutung hat,

$R^7$ bis $R^{10}$ die unter $R^2$ bis $R^5$ angegebene Bedeutung haben, wobei insgesamt von G2 nur eine freie Valenz zur Gruppe X ausgeht.

**2.** Gallensäurederivate der Formel I gemäß Anspruch 1, dadurch gekennzeichnet, daß G1 einen Rest der Formel II bedeutet

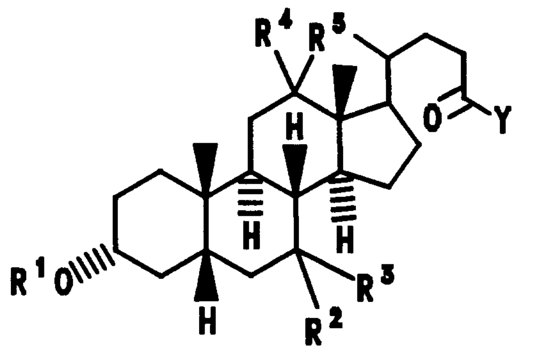

(II)

wobei

Y — OH, O-($C_1$-$C_4$)-Alkyl, $-NHCH_2COOH$,

$$-\underset{\substack{|\\ CH_3}}{N}CH_2COOH,$$

$-NHCH_2CH_2SO_3H$,

$$-\underset{\substack{|\\ CH_3}}{N}CH_2CH_2SO_3H$$

$R^1$ — H, Benzyl, Biphenylmethyl, Formyl, Acetyl,

$R^2$ bis $R^5$ — wobei $R^2$ und $R^3$ bzw. $R^4$ und $R^5$ jeweils gemeinsam der Sauerstoff einer Carbonylgruppe oder einzeln und jeweils unabhängig voneinander H, -OT, -NHT,

$$-O-\overset{\substack{O\\ \|}}{C}-T, \quad -NH-\overset{\substack{O\\ \|}}{C}-T,$$

-T bedeuten

wobei T

H, ein verzweigter oder unverzweigter ($C_1$-$C_4$)-Alkylrest oder eine freie Valenz zur Brückengruppe X darstellt, wobei von G1 insgesamt eine freie Valenz zur Bindung der Gruppe X ausgeht,

X — eine Bindung

$$-\underset{H}{N}-\ ,$$

$-CH_2CH_2NH-$

$-CH_2CH_2CH_2NH$-mit

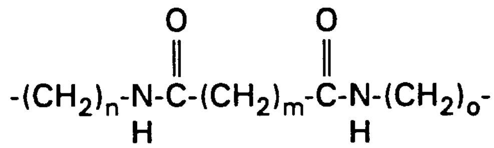

n = 2 oder 3, m = 1 bis 4, o = 2 oder 3 ist,

G2 einen Rest der Formel IV bedeutet

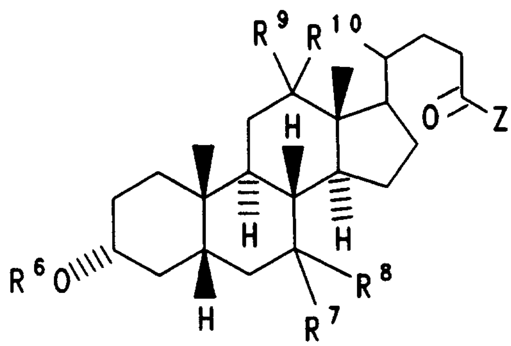

(IV)

wobei

| | |
|---|---|
| Z | eine freie Valenz zur Gruppe X ist oder die vorstehend unter Y angegebene Bedeutung hat, |
| $R^6$ | eine freie Valenz zur Gruppe X ist oder die vorstehend unter $R^1$ angegebene Bedeutung hat, |
| $R^7$ bis $R^{10}$ | die vorstehend unter $R^2$ bis $R^5$ angegebene Bedeutung haben, wobei von G2 nur eine freie Valenz zur Gruppe X ausgeht. |

**3.** Verfahren zur Herstellung von Gallensäurederivaten der Formel I gemäß Anspruch 1, dadurch gekennzeichnet, daß man
a) im Falle von X = Einfachbindung geeignete Formen von G1 und G2 nach im Prinzip bekannten Verfahren miteinander zur Reaktion bringt oder
b) im Falle von X = Brückengruppe
$\alpha$) reaktionsfähige Formen von G1-X mit G2 bzw.
$\beta$) reaktionsfähige Formen von G2-X mit G1
nach im Prinzip bekannten Verfahren miteinander zur Reaktion bringt oder
c) aus G1-X1 und X2-G2 nach bekannten oder soweit nicht bekannt, nach den unten näher beschriebenen Verfahren, Verbindungen der allgemeinen Formel I (G1-X-G2) herstellt, wobei X aus X1 und X2 entsteht durch Ausbildung einer kovalenten Bindung, insbesondere innerhalb einer Kondensations- oder Substitutionsreaktion.

**4.** Arzneimittel enthaltend ein Gallensäurederivat gemäß Anspruch 1.

**5.** Hypolipidaemikum enthaltend ein Gallensäurederivat gemäß Anspruch 1.

**6.** Verwendung eines Gallensäurederivates gemäß Anspruch 1 als Arzneimittel.